# EUROPEAN PATENT APPLICATION

(11) **EP 2 047 860 A1**
(43) Date of publication of application: **15.04.2009**
(21) Application number: 07291249.6
(22) Date of filing: 12.10.2007
(51) Int. Cl.: A61K 39/02, A61K 39/39, C12Q 1/18, G01N 33/53, G01N 33/569, A61P 31/06

(54) **Phamaceutical compositons comprosing actinomycete glycerol acyl derivatives antigens, their process of extractin, and their use against tuberculosis.**

(71) Applicant: CENTRE NATIONAL DE LA RECHERCHE SCIENTIFIQUE (CNRS), 75016 Paris (FR)
(72) Inventor: Puzo Germain, 31320 Auzeville Tolosane (FR); Layre Emilie, 31400 Toulouse (FR); Gilleron Martine, 31400 Toulouse (FR); Prandi Jacques, 31400 Toulouse (FR); De Libero Gennaro, 4103 Bottminge (CH); Stenger, Steffen, 89073 Ulm (DE)
(74) Representative: Colombet, Alain André

(57) **Abstract**

The present invention relates to the therapeutic use of actinomycete glycerol monomycolate derivatives as antigens, their process of extraction, and their use in the treatment or the prophylaxis of tuberculosis.

## Description

The present invention relates to the therapeutic use of actinomycete glycerol acyl derivatives antigens, their process of extraction and their use in the treatment or the prophylaxis of tuberculosis.

Each year, tuberculosis is the estimated cause of 2 million deaths. The causative agent of this disease is a bacterium, *Mycobacterium tuberculosis*, by which one of every three people is infected worldwide. It is transmitted through the air by sneezes or coughs of infected persons. The bacterium may be harboured in an inactive state by infected persons who will never develop the disease. However, under certain conditions, such as age or depression of the immune system, the bacterium may become active and cause the onset of active tuberculosis.

It seems that the mycobacterial envelope accounts for an important part of the virulence of bacteria of the *Mycobacterium* genus. Indeed, up to 40% of mycobacteria dry weight is constituted by lipids. These lipids have been shown to be involved in the modulation of the host immune responses. Some lipids are antigens which stimulate unconventional αβT cells in the context of CD1 proteins. The majority of mycobacterial lipid antigens are presented by the CD1 b isoform. This is the case for a large panel of structurally very different molecules, such as mycolic acids, glucose monomycolate, sulfoglycolipids and phosphatidyl-*myo-*inositol mannosides (PIM).

The existence of CD1-restricted T cells specific for several of the most abundant mycobacterial lipids strongly suggests that CD1 proteins are important in the host response to *M*. *tuberculosis.*

At present, there are two ways of fighting tuberculosis: antibiotic therapy and vaccination.

The vaccine used for the prophylaxis of tuberculosis consists of a live attenuated bacterium of the *Mycobacterium bovis* species. It is named BCG, Bacillus of Calmette and Guerin, after the two scientists who first devised it, at the beginning of the 20^{th} century. The different strains of BCG currently used for vaccination are notably described in Behr M.A. et al. Science (1999) 284:1520-1523.

In addition to its flaw as being a live vaccine, which precludes its use in immunodepressed patients, its protection against tuberculosis is controversial. Thus, BCG protective efficacy in adults range from 0% to 80% according to varying studies, besides, it fails to protect against pulmonary tuberculosis, the most prevalent disease form in adults.

Moreover, antibiotic resistant strains of *M*. *tuberculosis* have been found in over 35 countries.

Hence, it is a subject of the present invention to provide a more effective way of treating and/or preventing tuberculosis.

It is thus highly desirable to provide further new immunogenic compounds.

The invention notably provides new antigens, which have been shown to stimulate CD1-restricted T lymphocytes *in vitro*. Said antigens are glycerol-monomycolate derivatives and have been isolated from actinomycetes, such as mycobacteriae.

The characterization of the mycolic acid derivatives has been largely reported (Laval et al., Anal Chem 2001, 73(18):4537-4544; Villeneuve M. et al., Biochim Biophys Acta 2005, 1715(2):71-80; Villeneuve M. et al., Biochim Biophys Acta 2007, 1768(7):1717-1726; Watanabe M. et al., Microbiology 2001, 147(Pt 7):1825-1837).

These compounds are known although their therapeutic use, and this is one object of the present invention, has neither been disclosed nor suggested. Additionally, the corresponding acylated forms and reduced forms thereof as well as the specific *(R)* enantiomer of such compounds are novel, according to a further object of the present invention.

More particularly, the invention relates to the compounds of the following general formula (I): 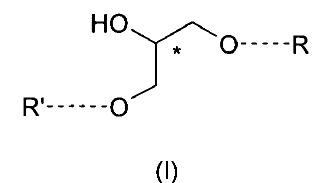 wherein:
R is a residue of a α-alkylated, β-hydroxylated carboxylic acid comprising 20 to 90 carbon atoms;
R' represents a hydrogen atom or acyl group (-C(=O)-Alkyl) where Alkyl comprises 1 to 10 carbon atoms; and
   * denotes an asymmetric carbon atom;
and its enantiomers, diastereoisomers, mixtures thereof, and their therapeutic uses.

The asymmetric carbon of the glycerol skeleton (*) preferably exhibits a *(R)* configuration.

Preferably, R is of formula: 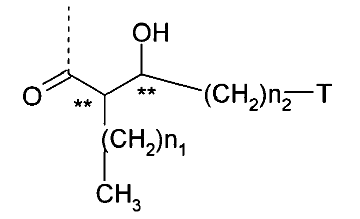 where (I) is attached to R through ---- ; and
T represents a methyl group or a branched or linear C10-C60 hydrocarbon chain which optionally comprises one or more double bond(s), -(C=O)- group, cyclopropyl and/or epoxy, and/or is optionally substituted with one or more groups chosen from OH, OMethyl;
** denotes an asymmetric carbon;
n1, n2 identical or different, independently represent an integer.

Preferably, n1 is an integer comprised between 5 and 40; more preferably 5 to 30;
Preferably, n2 is an integer comprised between 5 and 30; more preferably 10 to 20;
Preferably, both **C asymmetric carbons exhibit the *(R)* configuration.
Preferably, T is chosen from a methyl group or a branched or linear C10-C60, preferably C15-C50 hydrocarbon chain which optionally comprises one to five double bond(s), and/or represents a group of formula: 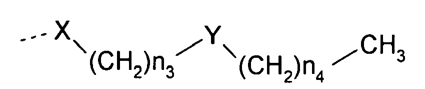 where
X is chosen from: 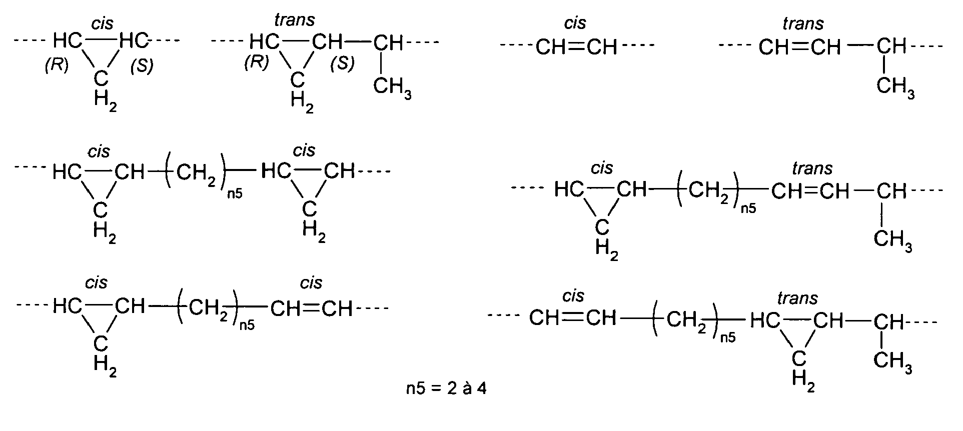 Y is chosen from: 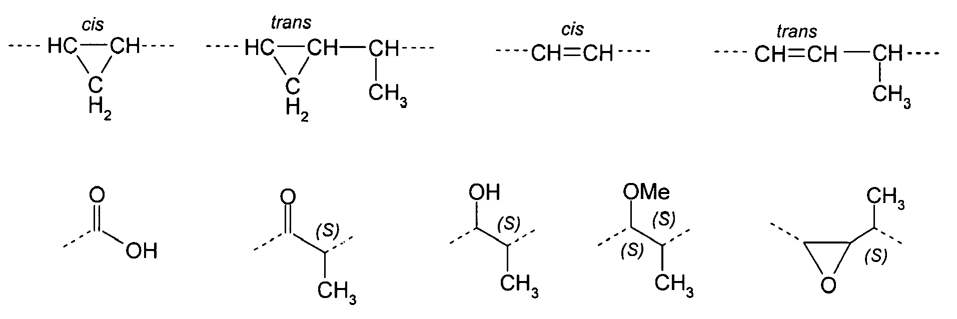 and n3, n4 identical or different, independently represent an integer comprised between 5 and 30; preferably n3 represents an integer comprised between 12 to 16 and n4 is chosen from 11, 13, 15, 17, 18 and 19,
provided that when Y is ---C(=O)-OH, then T is ---(CH2)ₙ₃-X-C(=O)-OH.

Mycolic acid derivatives are characterized by Laval et al., Anal Chem 2001, 73(18):4537-4544; Villeneuve M. et al., Biochim Biophys Acta 2005, 1715(2):71-80; Villeneuve M. et al., Biochim Biophys Acta 2007, 1768(7):1717-1726; Watanabe M. et al., Microbiology 2001, 147(Pt 7):1825-1837.

According to a preferred embodiment, the present invention concerns *per se* the compounds of formula (I) as defined above where R' represents a
-C(=O)-Alkyl group.
Such compounds may be obtained from the corresponding compounds of formula (I) as described above by application or adaptation of any known acylation methods generally used, in particular by reacting acetic anhydride.

According to another preferred embodiment, the present invention concerns *per se* the compounds of formula (I) as defined above where T represents: 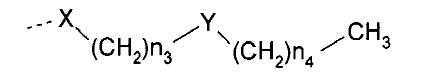 where Y is

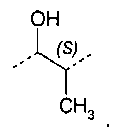

The corresponding compounds are herein referred to as "reduced compounds".
Such compounds may be obtained form the corresponding compounds of formula (I) as described above by application or adaptation of any known reduction methods generally used. Preferably, this reaction may be carried out by means of NABH₄ treatment.

According to another preferred embodiment, the present invention concerns *per se* the compounds of formula (I) as defined above where the C* exhibits the (R) configuration.

GroMM herein refers to glycerol monomycolate.

According to a further embodiment, the invention relates to a pharmaceutical composition comprising a compound of formula I such as defined above.
The invention further relates to a pharmaceutical composition characterized in that it comprises a mixture of compounds selected from the compounds of formula I.

The invention also relates to a pharmaceutical composition comprising at least one compound as defined above, or a composition as defined above, in association with a pharmaceutically acceptable vehicle.
Said pharmaceutical compositions are for example vaccines.

Further, the invention also relates to a combination of one or more compound(s) of formula I with one or more mycobacterial lipids, such as compound(s) chosen from the group consisting in mycobacterial phospholipids, mycobacterial lipoglycans, mycobacterial glycolipids and phospholipids, as well as the pharmaceutical composition comprising the same.
Such lipids are known in the art and were disclosed in Nigou et al. (2003) Biochimie, 85, 153-166.
It has been unexpectedly discovered that the above combinations exhibit a synergistic activity, as apparent from the results below.

A pharmaceutically acceptable carrier comprises in particular liposomes.

Said pharmaceutical compositions are useful for treating and/or preventing affections by actinomycetes, in particular mycobacteriae, such as tuberculosis or cutaneous affections.
The composition may also comprise vaccine adjuvants. Vaccine adjuvants for use in human individuals or animals are well known to the man skilled in the art, a list of such salts can be found for instance in "A compendium of vaccine adjuvants and excipients" 2^{nd} edition, Vogel *et al*. Particular vaccine adjuvants notably comprise aluminum salts or M59, for example.
The invention particularly relates to a pharmaceutical composition as defined above, **characterized in that** it is presented in a form intended for administration by oral or injectable route.
The invention more particularly relates to a pharmaceutical composition as defined above, **characterized in that** it comprises one or more other products useful for the treatment or the prophylaxis of tuberculosis, such as BCG or mycobacterial proteins or actinomycete lipids.

The expression "actinomycete glycerol acyl derivatives" refers to lipids found in bacteria of the *Cornybacterium, Mycobacterium*, and *Nocardia* (CMN) genus, and preferably in *Mycobacterium bovis* BCG, *Mycobacterium tuberculosis, Mycobacterium smegmatis, Mycobacterium gastri, Mycobacterium marinum, Mycobacterium fortuitum, Mycobacterium xenopi, Mycobacterium kansasii, Cornybacterium glutamicum* and *Nocardia asteroids*. According to a preferred embodiment said lipids are antigens of *M*. *bovis* BCG.
Other products useful for the treatment or the prophylaxis of tuberculosis notably comprise immunomodulators, such as cytokines, DNA fragments encoding *M*. *tuberculosis* antigens, live *M*. *tuberculosis* deletion mutants, such as mutants in which virulence genes have been deleted, or live recombinant BCG, such as BCG expressing antigens of *M*. *tuberculosis.*
As used herein, tuberculosis refers to the disease caused in humans by the bacterium *Mycobacterium tuberculosis*, and also to the corresponding disease in animals.

According to another embodiment, the invention relates to products comprising:
- at least one compound as defined above, and
- at least one other product useful for the treatment or the prophylaxis of tuberculosis, such as BCG or mycobacterial proteins,
   as a combined preparation for simultaneous, separate or sequential use in the treatment or the prophylaxis of tuberculosis.
The invention also relates to the use of at least one compound as defined above, or of an above mentioned composition, for the preparation of a medicament, notably a vaccine, intended for the treatment or the prophylaxis of tuberculosis.
The invention also relates to a compound as defined above as a medicament for the treatment or the prophylaxis of tuberculosis.

Optionally, the vaccine may comprise a vaccine adjuvant such as described above.

The invention relates to the use of at least one compound as defined above, or of a composition according as defined above, as an immune reaction activator, and more particularly an inflammatory reaction activator.
By "immune reaction activator" is meant a compound which has the ability to activate components or processes of the immune reaction, *in vitro* or *in vivo*, in particular cells of the immune system, such as T lymphocytes, B lymphocytes, antigen presenting cells (APCs), such as dendritic cells or macrophages, monocytes or granulocytes.
By "inflammatory reaction activator" is meant a compound which has the ability to activate components or processes of the inflammatory reaction, *in vitro* or *in vivo*, such as diapedesis, capillary permeabilization, macrophage activation or fever onset for instance.

The invention also relates to the use of at least one compound as defined above, or of a composition as defined above, to induce the activation of T lymphocytes, notably CD1-restricted T lymphocytes. The activation can proceed in *vitro* or *in vivo*.
The activation of T lymphocytes can be assessed by several methods, such as measuring cell proliferation or cytokine production such as IFN-γ (interferon-γ), IL-2 (interleukine-2), IL-4 (interleukine-4), or TNF-α (tumor necrosis factor α), or GM-CSF for instance.
CD1-restricted T lymphocytes are T lymphocytes which are activated by antigens presented by CD1 molecules.
The invention also relates to the use of at least one compound as defined above, or of a composition as defined above, to induce the production of cytokines such as IFN-γ, TNF-α, IL-2, IL-4, or GM-CSF.
This production induction can be done *in vitro* or *in vivo*. The production of cytokines such as IFN-γ, TNF-α, IL-2, IL-4 or GM-CSF can be measured for instance by immunoassays, such as ELISA (enzyme linked immunosorbent assay) or EIA (enzyme immunoassay).

According to another embodiment, the invention relates to a process for generating a T cell clone specific to the compounds of formula (I) , **characterized in that** it comprises the following stages:
1) incubating antigen presenting cells (APCs), notably dendritic cells, with an actinomycete lipidic preparation, with the exception of *Mycobacterium tuberculosis* lipidic preparation, or a compound of formula (I),
2) contacting peripheral blood mononuclear cells with the envelope antigen loaded APCs to obtain proliferating T cells,
3) cloning proliferating T cells by limiting dilution and selecting the clone releasing a molecule selected from the group comprising cytokines such as IFN-γ, TNF-α, GM-CSF, IL-2 or IL-4 when contacted by a compound of formula (I).

Preferably, in step 1), the actinomycete lipidic preparation is a *Mycobacterium bovis* BCG lipidic preparation.
The invention further relates to T cell clones such as obtained by the above-mentioned process. Such a T cell clone is described in the examples as Z5B71.

According to another embodiment, the invention relates to a process for screening products, such as actinomycete glycerol acyl derivatives extracted from actinomycetes, such as *Mycobacterium*, *Corynebacterium* and *Nocardia*, **characterized in that** it comprises the following stages:
- contacting dendritic cells loaded with the product to screen, notably actinomycete glycerol acyl derivatives extracted from *Mycobacterium bovis* BCG, with the above-defined T cell clones,
- detecting a molecule selected from the group comprising cytokines, such as IFN-γ, TNF-α, GM-CSF, IL-2 or IL-4, released by the T cell clones.

The invention also relates to a process for the extraction of compounds as defined above, or of a composition as defined above, from actinomycetes, such as *Mycobacterium*, *Corynebacterium* and *Nocardia* **characterized in that** it comprises the following stages:
a. treatment of the bacteria with a mixture of methanol and chloroform to obtain a chloroform/methanol extract,
b. concentration of the chloroform/methanol extract followed by its partition between a chloroform phase (herein called the lipidic fraction) and an aqueous phase,
c. taking off the lipidic fraction.

According to a preferred aspect, said process further comprises the following additional steps:
d. evaporation of most of the chloroform, followed by addition of acetone thereto to obtain a precipitate and a soluble acetone phase,
e. taking of the acetone insoluble phase followed by its concentration, followed by addition of methanol to obtain a precipitate and a methanol insoluble phase,
f. taking of the methanol insoluble phase, followed by its concentration and application of the concentrated methanol insoluble phase on a silicic acid column,
g. elution of a fraction from the above-mentioned silicic acid column by chloroform, methanol and mixtures thereof, said fraction corresponding to a composition as defined above,
h. if necessary purification of the fraction eluted from the silicic acid column to obtain different preparations respectively containing substantially only one of the compounds as defined above.

The present invention also concerns the compositions obtainable by the above process as well as the pharmaceutical compositions comprising the same, and the use of said composition for the preparation of a medicament for the prophylaxis or the treatment of tuberculosis, the use of said composition as an immune reaction activator, the use of said composition to induce the activation of T lymphocytes, the use of said composition to induce the production of cytokines such as IFN-γ, TNF-α, IL-2, IL-4, or GM-CSF as defined above.

### BRIEF DESCRIPTION OF THE DRAWINGS

**Figure 1** represents the antigenic potency of lipidic fractions from various bacterial species of the CMN group. Stimulation of the Z5B71 T cell clone by lipidic fractions measured by IFN-γ ELISA. Bars indicated SD.
   *Corynebacterium glutamicum* (■), Nocardia (●), *M. bovis* BCG (▲), *M*. *tuberculosis* H37Rv (◆), *M*. *smegmatis* (□), *M*. *fortuitum* (○), *M*. *tuberculosis* H37Ra (Δ)
**Figure 2** represents the fractionation of the *M*. *bovis* BCG "Methanol insoluble" lipid pool and the reactivity of the different fractions obtained with the Z5B71 T cell clone.
   TLC analysis of eight representative fractions (fractions 1, 3, 5, 10, 18, 19, 28 and 34) obtained by silica chromatography purification of "methanol insoluble" fraction is presented in (A). The encircled compound corresponds to the active compound. The same fractions were tested for stimulation of the Z5B71 T cell clone by measuring IFN-γ release (B). Bars indicated SD.

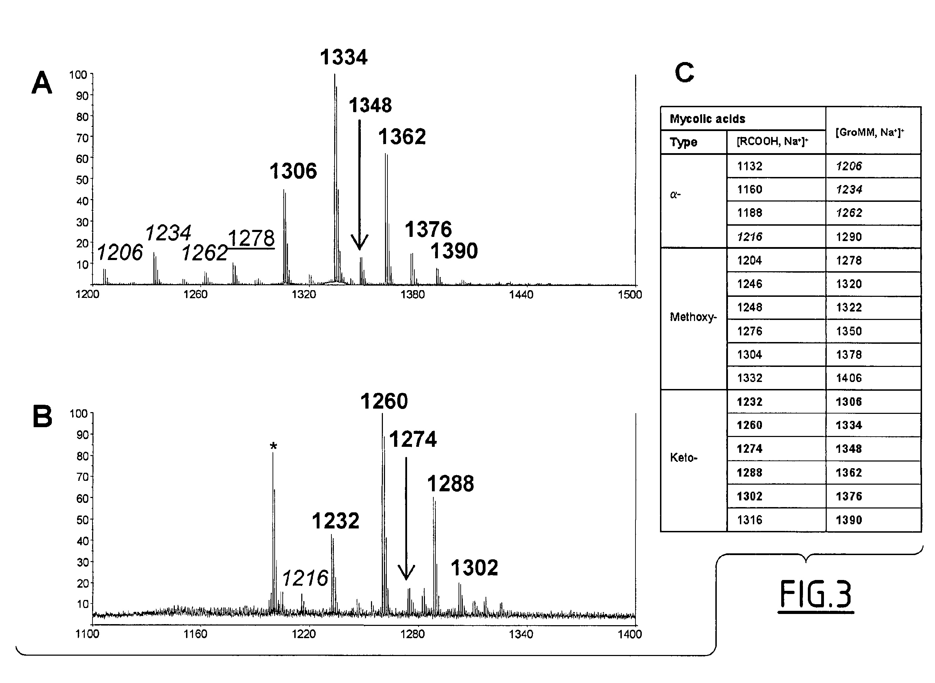

**Figure 3** represents the MALDI-*Tof*-MS analysis in positive mode of *M*. *bovis* BCG GroMM (A) and of the mycolic acids obtained after saponification of *M*. *bovis* BCG GroMM (B). The asterisk corresponds to a contaminant. The table presented in (C) details the calculated masses of sodium adducts of the different expected mycolic acids types and of corresponding GroMM.

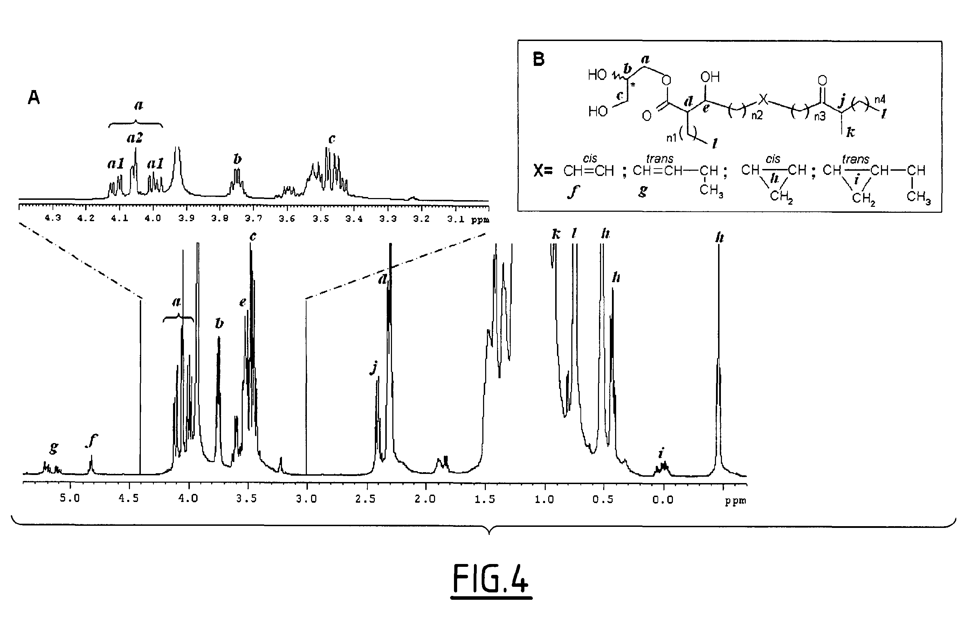

**Figure 4** represents the structure determination of *M*. *bovis* BCG GroMM by ¹H NMR analysis (δ ¹H: -0.7 - 5.4) (A). Specific GroMM protons signals are annotated *a* to *k* and are assigned on the structure of the major forms of *M*. *bovis* BCG GroMM, i.e. esterified by ketomycolic acids, presented in (B). n1 = 19, 21 or 23 ; n2 et n4 = 15, 17 or 19 ; n3= 12 to 17.

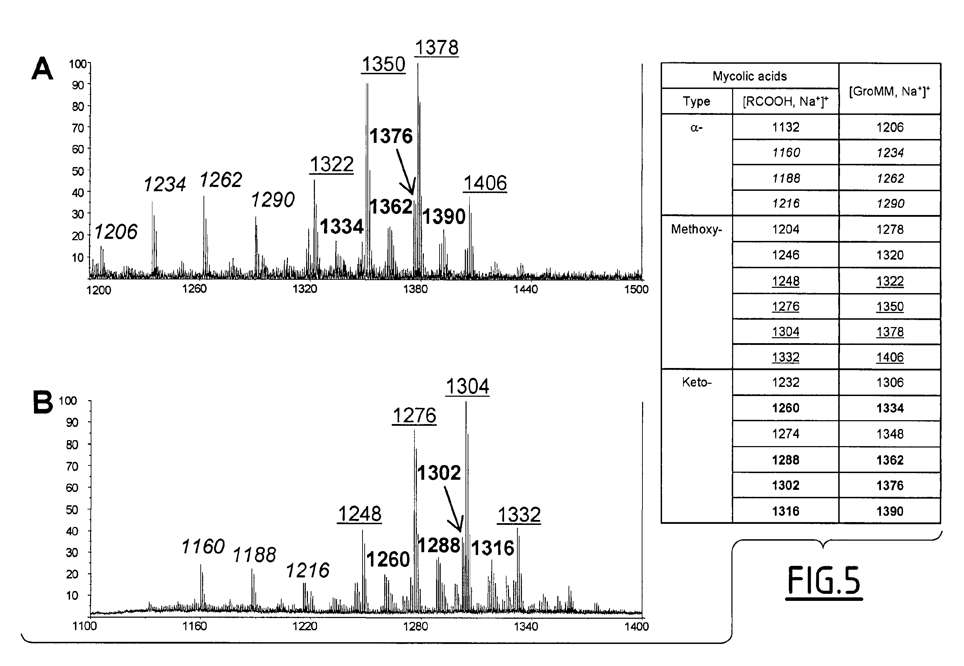

**Figure 5** represents the MALDI-*Tof*-MS analysis in positive mode of synthetic GroMM (A) and of the *M*. *tuberculosis* H37Rv mycolic acids used for the synthesis (B). The table presented in (C) detailed the calculated masses of sodium adducts of the different expected mycolic acids types and of corresponding GroMM.

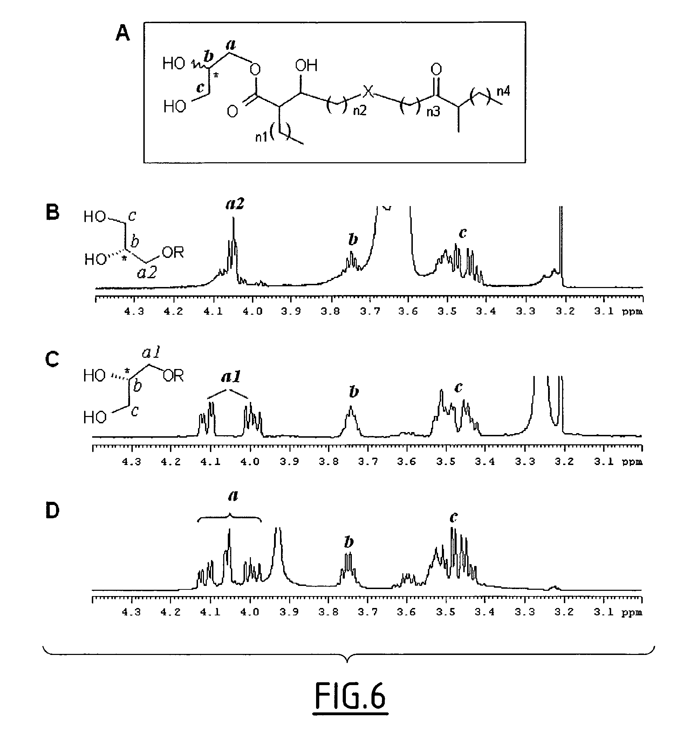

**Figure 6** represents the structure determination of synthetic GroMM by ¹H NMR analysis (δ ¹H: 3.0 - 4.4) (B and C) compared to the *M*. *bovis* BCG GroMM (D). The structure of the major forms of *M*. *bovis* BCG GroMM, i.e.esterified by ketomycolic acids, presented in (A). n1 = 19, 21 or 23 ; n2 et n4 = 15, 17 or 19 ; n3= 12 to 17 and glycerol protons signals are annotated *a* to *c*.
   The structures presented in (B) and (C) correspond to (S)-1-O-mycoloyl-glycerol (1-O-mycoloyl-sn-glycerol) and (R)-1-O-mycoloyl-glycerol (3-O-mycoloyl-sn-glycerol), respectively. R corresponds to mycolic acid.

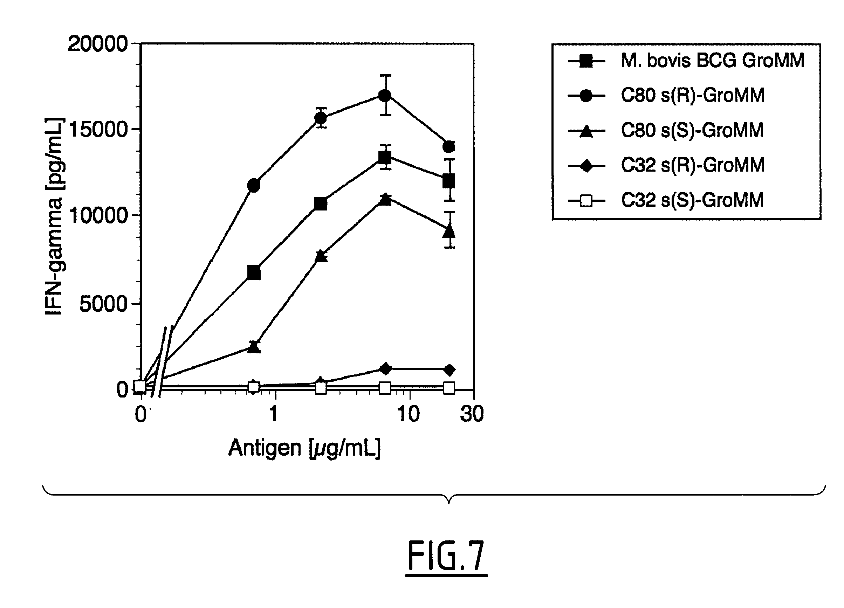

**Figure 7** represents the biological activities of hemi-synthetic GroMM. In C32 or C80 s(S)- or s(R)- GroMM, C32 corresponds to corynomycolic acids and C80 to mycolic acids while (S) corresponds to (S)-1-O-mycoloyl-glycerol (1-O-mycoloyl-*sn*-glycerol) and (R) to (R)-1-O-mycoloyl-glycerol (3-O-mycoloyl-*sn*-glycerol). Release of IFN-γ by the Z5B71 T cell clone was measured by ELISA. Bars indicate SD.

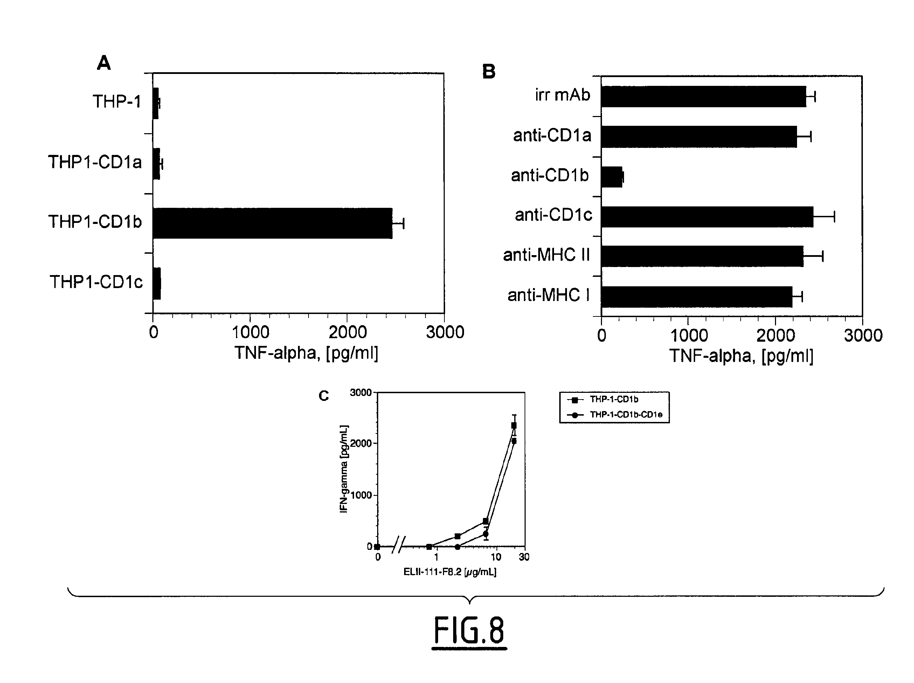

**Figure 8** represents the CD1b-restricted and CD1e-independent presentation of GroMM, using THP-1 transfected cells (A) and CD1a to c antibodies (B). In (C), THP-1-CD1b (■) and THP-1-CD1b-CD1e (●) cells were used. Release of TNF-α (A and B) or IFN-γ (C) by the Z5B71 T cell clone was measured by ELISA. Bars indicate SD.

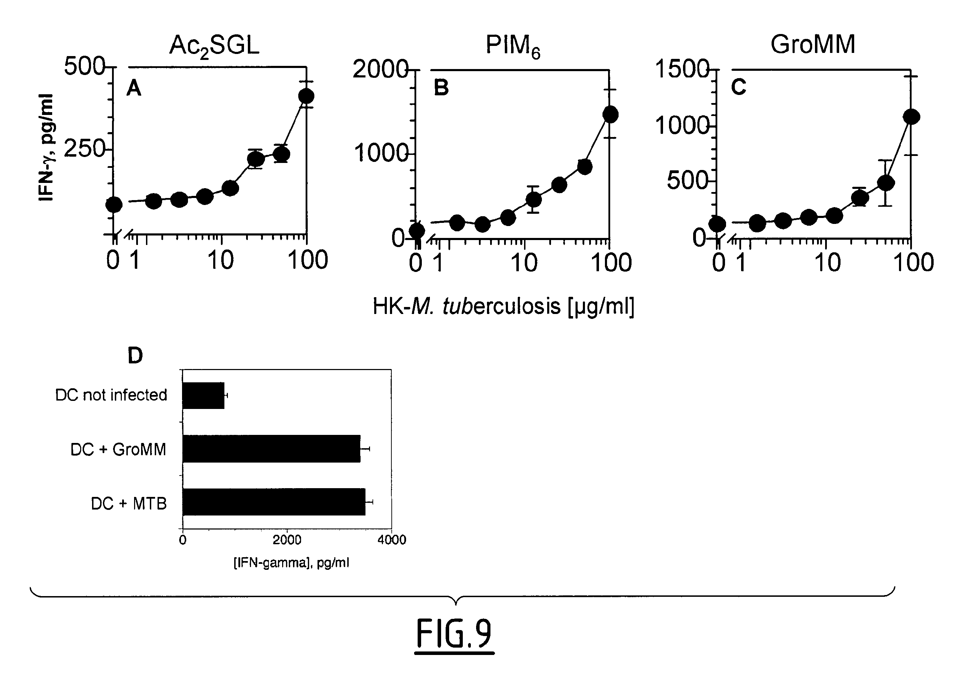

**Figure 9** represents the recognition of DCs pulsed with heat killed *M*. *tuberculosis* by the CD1b-restricted T cells specific to Ac₂SGL (A) , PIM₆ (B) and GroMM (C) and infected with living *M*. *tuberculosis* (D). Release of IFN-□ by the Z5B71 T cell clone was measured by ELISA. Bars indicate SD.

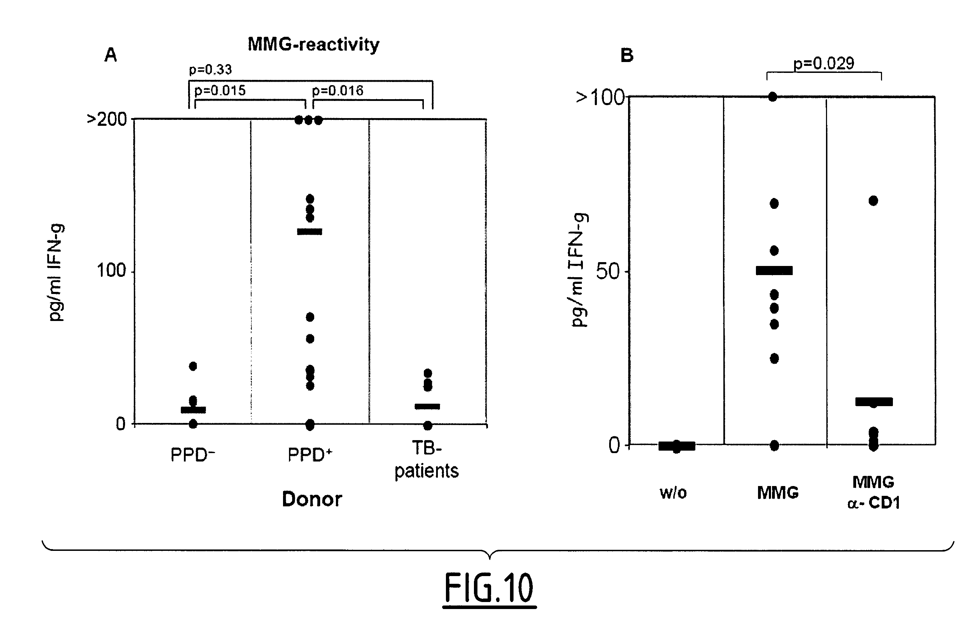

**Figure 10** represents the reactivity to GroMM of PPD-negative and PPD-positive donors: PBMCs of human donors were stimulated with GroMM in the presence of autologous, CD1 expressing DCs. After six days, culture supernatant was collected and IFN-γ concentration determined by ELISA: (A) The reactivity to GroMM was compared between naïve (n=10), BCG immunized (22) or latently infected healthy donors (n=19) and TB-patients (n=11). (B) To investigate the restriction element, GroMM reactivity was tested in the presence or absence of a CD1b blocking antibody in ten PPD-positive healthy donors. Black circles show individual donors, horizontal bars represent the average for each group.

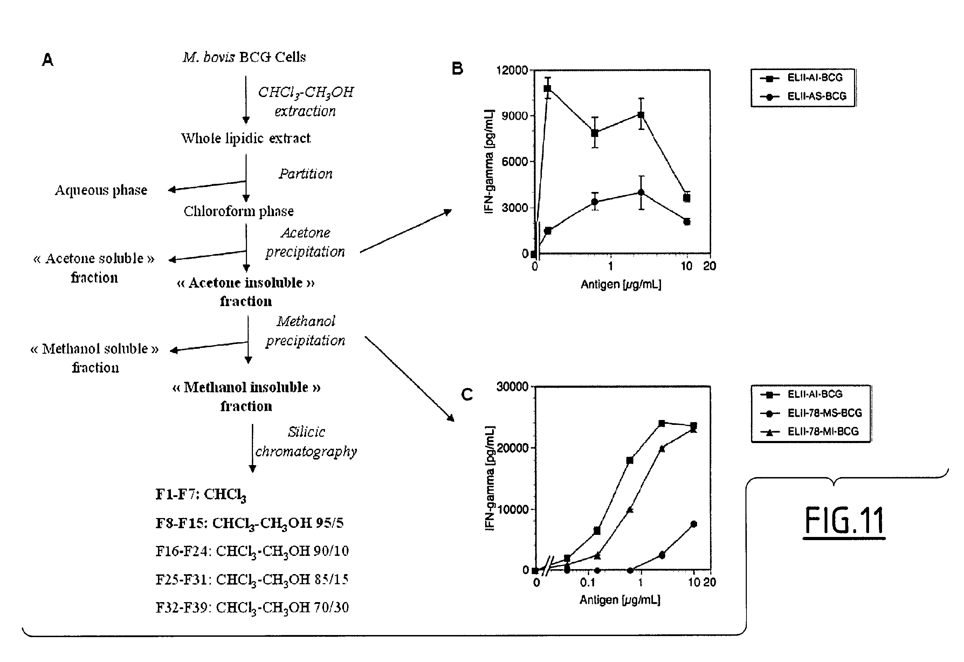

**Figure 11** represents the purification scheme of GroMM from *M*. *bovis* BCG (A). The purification was followed by antigenic potency of the lipidic fractions (B and C). Released IFN-γ by the Z5B71 T cell clone was measured by ELISA. Bars indicate SD. The lipidic fraction refers to the chloroform phase.

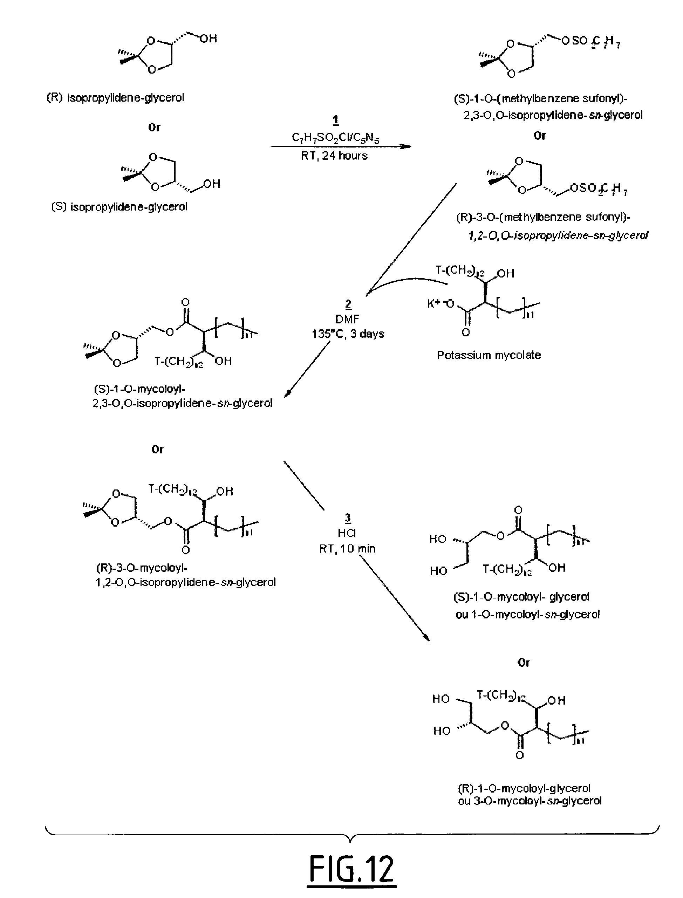

**Figure 12** represents the synthesis scheme of (S)-1-O-mycoloyl-glycerol (1-O-mycoloyl-*sn*-glycerol) and (R)-1-O-mycoloyl-glycerol (3-O-mycoloyl-sn-glycerol).

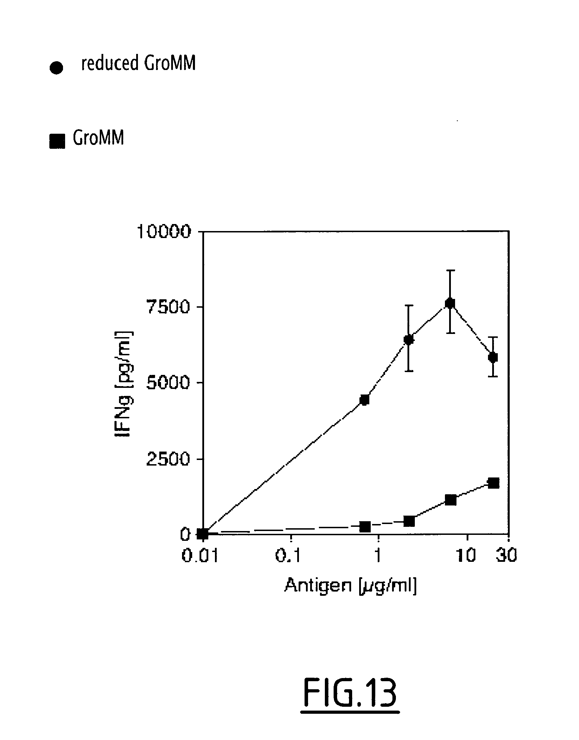

**Figure 13** represents the biological activities of the mycobacterial product obtained from *M*. *bovis* BCG and of the corresponding reduced product obtained by reduction.

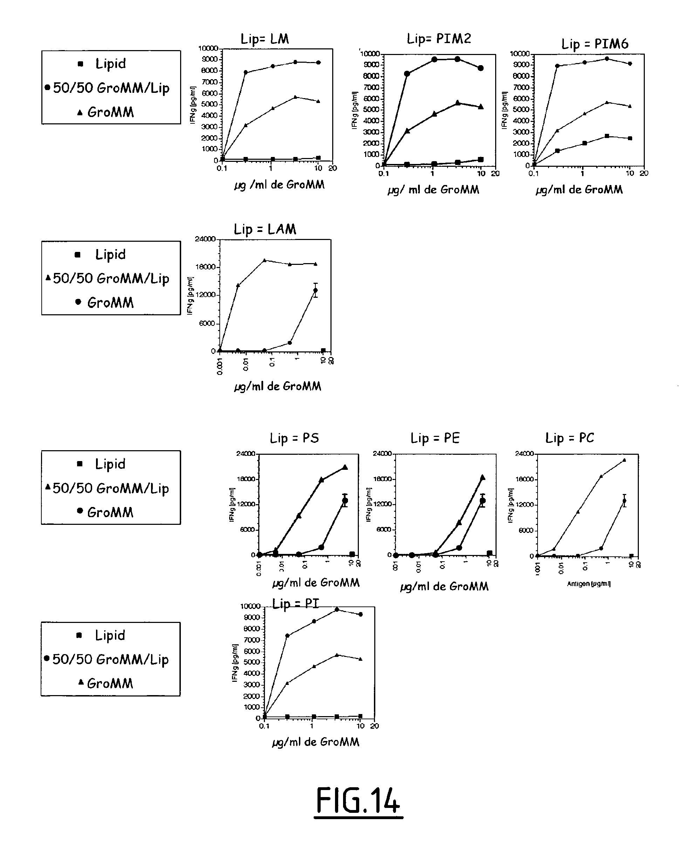

**Figure 14** represents the biological activities of the mixtures of the mycobacterial product obtained from BCG with various lipids. Mycobacterial lipids: LAM (lipoarabinomannan), LM (lipomannan), PIM₂ and PIM₆ (phosphatidyl-*myo-*innositol-mannosides comprising 2 or 6 mannose units). Phospholipids: PS: phosphatidyl serine, PE: phosphatidyl ethanolamine, PC: phosphatidyl choline, PI: phosphatidyl inositol.

### EXAMPLES

### Materials and Methods

**Reagents.** L-1,3-O-isopropylidene-sn-glycerol and D-1,2-O-isopropylidene-*sn-*glycerol, glyceryl tripalmitate, 1,2 dipalmitoyl-sn-glycerol and monopalmitoyl glycerol were purchased from Sigma. Cardiolipids were purified from *M*. *bovis* BCG lipidic extract, as described in Gilleron et al. (2001) J Biol Chem 276, 34896-904.

**Bacterial Strain and Culture Conditions.** *Nocardia asteroides* and *Corynebacterium glutamicum* were grown in suspension during 2 to 3 days at 32°C on Luria Broth and Brain Heart Infusion medium, respectively. Mycobacteria were grown during 4 to 8 weeks at 37°C on Sauton's medium as surface biofilm. Cells were harvested, separated from the culture media, and left in chloroform/methanol (2:1, v/v) at room temperature in order to kill bacteria.

**Cell Culture.** Human promyelocytic THP-1 were transfected with cDNA of human CD1A, or CD1 B, or CD1 C or double-transfected with CD1B and CD1 E using the BCMGS-Neo and -Hygro vectors by electroporation. Cells were grown in RPMI-1640 supplemented with 10% fetal calf serum (FCS), 10 mM HEPES, 2 mM UltraGlutamine II, MEM nonessential amino acids, 1 mM Na-pyruvate (all from Cambrex, Brussels, Belgium) and 100 µg/ml kanamycin (Invitrogen, Basel, Switzerland). DCs were isolated from peripheral blood mononuclear cells (PBMCs) of healthy donors by culturing in the presence of recombinant IL-4 and GM-CSF, as described previously Porcelli et al. (1992), Nature 360, 593-7. T cell clones were generated as described De Libero (1997), ed. Butcher, N. F. a. G. (IRL, Oxford), pp. 123-40, and grown in RPMI-1640 supplemented with 5% AB human serum (Swiss Red Cross), 10 mM HEPES, 2 mM UltraGlutamine II, MEM nonessential amino acids, 1 mM Na-pyruvate, 100 µg/ml kanamycin and 100 units/ml human recombinant IL-2.

**T Cell Activation Assays.** DCs (3 x 10⁴/well) or CD1-transfected THP-1 cells (3 x 10⁴/well) in RPMI-1640 medium containing 10% FCS were incubated for 2 hr at 37°C with different concentrations of sonicated antigens before the addition of T cells (10⁵/well in triplicate). Supernatants were harvested after 36 hr of incubation, and cytokine release was measured by using enzyme-linked immunosorbent assay (ELISA) kits (IFN-γ from Instrumentation Laboratory, Schlieren, Switzerland; TNF-α from BD Pharmingen, Basel, Switzerland). Data are expressed as mean pg/ml ± standard deviation (SD) of triplicates.

**Analysis of CD1 Restriction.** CD1 restriction was investigated by inhibiting T cell activation using the following monoclonal antibodies (mAb) at 10 µg/ml 30 min before the addition of T cells: OKT6 (anti-CD1a, American Type Culture Collection ATCC CRL-8019), WM-25 (anti-CD1b; Immunokontakt, Lugano, Switzerland), L161 (anti-CD1c; Instrumentation Laboratory), W6-32 (anti-MHC class I, ATCC) and L243 (anti-MHC class II, ATCC). Anti-TCRVγ9 (B3) mAb was used as irrelevant mAb.

**Pulsing and infection of DCs with M.** ***tuberculosis.** M. tuberculosis* was killed at 80°C for 20 min and incubated in RPMI-1640 medium containing 10% FCS for 2 hours at 37°C with DCs (3 x 10⁴/well) before addition of T cells (10⁵/well in triplicate). DCs were infected as previously described De Libero et al. (2005) Immunity 22, 763-72 and IFN-γ release was monitored by ELISA.

**Lipidic Fractions Used for Generation of T Cell Clone.** The Z5B71 T cell clone was generated with a M. *bovis* BCG PIM₂-enriched fraction, obtained previously Gilleron et al. (2001) J Biol Chem 276, 34896-904. Briefly, *M. bovis* BCG cells were suspended in chloroform/methanol (1:1, v/v) and filtered four times. The chloroform/methanol extract was concentrated and constituted the lipidic fraction, which was further partitioned between water and chloroform. The chloroform phase was evaporated and suspended in a minimum volume of chloroform. The addition of acetone overnight at 4°C led to formation of a precipitate, which was centrifuged (3,000 g at 4°C for 15 min) to generate an "acetone-soluble" phase and an "acetone-insoluble" phase. The "acetone-soluble" phase was applied to a QMA-Spherosil M (BioSepra SA, Villeneuve-la-Garonne, France) column eluted successively with chloroform, chloroform/methanol (1:1, v/v), methanol to elute neutral compounds and then with chloroform/methanol (1/:2, v/v) containing 0.1 M ammonium acetate to elute negatively charged compounds (as PIM₂).

**Lipidic fractions from several actinomycetes.** Different lipidic fractions were prepared (as described above) from several actinomycetes species and tested against the Z5B71 T cell clone: the lipidic fraction from *M*. *smegmatis, M*. *tuberculosis* H37Ra, *M*. *fortuitum*, *M*. *chelonae*, *M*. *xenopi*, *N*. *asteroides* and *C*. *glutamicum*, the "acetone-soluble" phase from *M*. *smegmatis*, *M*. *tuberculosis* H37Rv, *M*. *gastri*, *M*. *kansasii*, *M*. *marinum* and *M*. *bovis* BCG and the "acetone-insoluble" phase from *M*. *tuberculosis* H37Rv, *M*. *gastri*, *M*. *kansasii*, *M*. *marinum* and *M*. *bovis* BCG.

**Purification of the GroMM from *M*. *bovis* BCG.** The "acetone-insoluble" fraction from *M. bovis* BCG, obtained as described above, was subjected to methanol precipitation giving "methanol soluble" and "methanol insoluble" fractions. The "methanol-insoluble" fraction (150 mg) was fractionated on a silica acid column (1.3 x 23 cm) eluted with 7x175 ml of chloroform (fractions 1-7), 8x200 ml of chloroform-methanol (95:5, v/v) (fractions 8-15), 9x225ml of chloroform - methanol (9:1, v/v) (fractions 16-24), 7x175 ml of chloroform-methanol (85:15, v/v) (fractions 25-31) and 7x175 ml of chloroform-methanol (7:3, v/v) (fractions 32-38). Fractions 5 to 8 were found to contain GroMM and were pooled.
Purifications were checked by TLC on aluminium-backed silica gel plates (Alugram Sil G; Macherey-Nagel) using as migration solvent chloroform/methanol (95:5, v/v). Anthrone (9,10-dihydro-9-oxo-anthracene) (Sigma) at 0.2% dissolved in H₂SO₄ at 85% was used to detect glycolipids.

**GroMM Acetylation.** Peracetylation was realized on 200 µg of GroMM which were dissolved in acetic anhydride/anhydrous pyridine (1:1, v/v) and heated at 80°C for 30 min. The reaction mixture was dried under a stream of nitrogen and dissolved in chloroform for MALDI-*Tof*-MS analysis. For selective acetylation of GroMM hydroxyl functions, 16 µl of acetic anhydride were added to 10 mg of GroMM dissolved in 150µl of pyridine and kept 3 hours at 0°C. Mono- and di-acetylated GroMM were extracted by CHCl₃ and purified on silica column eluted successively by CHCl₃ and CHCl₃/CH₃OH 95/5 (v/v). Number of acetyl substituents and their location on GroMM were determined by MALDI-*Tof*-MS and NMR analysis.

**GroMM Saponification.** 800 µl of methoxyethanol/KOH 20% (7:1, v/v) were added to 500 µg of GroMM. The mixture was then heated in an oven at 100°C for 3 h. After cooling and acidification by aqueous sulphuric acid pH 3, mycolic acids were extracted three times by diethyl ether (v/v). Etheral phases were pooled, washed five times with water and dried under a stream of nitrogen. Mycolic acids were redissolved in 100 µl chloroform for MALDI-*Tof*-MS analysis.

**GroMM Reduction.** 1 mg of GroMM purified from BCG was reacted with 1,3 mg of NaBH₄ in 140 µl THF, under stirring for 15 minutes at room temperature. Reaction was stopped by adding acetic acid. The reaction mixture was evaporated, stirred with dichloromethane and washed twice with water.

**GroMM Synthesis.** Reactions were performed under argon in anhydrous solvents.
*1-*O*-(4-Methylbenzenesulfonyl)-2,3-*O,O*- isopropylidene-sn-glycerol* (Sowden et al. (1942) J Am Chem Soc 42, 1291-3) : p-toluenesulfonyl chloride (0.3 g, 1.57 mmol) was added at 0°C to 2,3-*O*,*O*- isopropylidene-*sn*-glycerol (230 mg, 1.74 mmol) dissolved in anhydrous pyridine (0.8 ml). After 24 h at room temperature, diethyl ether was added to the reaction mixture and the organic phase was washed sequentially with cold, 1 M hydrochloric acid, water, saturated sodium bicarbonate solution and water. The ether extract was dried on magnesium sulphate and concentrated. Chromatography on silica gel (elution with petroleum ether / ethyl acetate 4/1, v/v) gave the product (around 100 mg, 0.35 mmol). In the same manner, 3-*O*-(4 methylbenzenesulfonyl)-1,2-*O*,*O*-isopropylidene-sn-glycerol was obtained from 1,2-*O*,*O*- isopropylidene-*sn*-glycerol.

*Potassium mycolates:* Mycolic acids (gift from M. A. Lanéelle, IPBS, Toulouse) were released from *M*. *tuberculosis* H37Rv cells according to the method described in Laval et al. (2001) Anal Chem 73, 4537-44. Potassium mycolates were obtained by two successive washings of mycolic acids dissolved in chloroform with 10 mM hydrochloric acid and 5 M potassium hydroxide. After extraction with chloroform, potassium mycolates were dried on magnesium sulphate and concentrated.

*1-*O*-Mycoloyl-2,3-*O,O*- isopropylidene-sn-glycerol* (Defaye et al. (1956) Bull Soc Chim Biol (Paris) 38, 1301-4): Potassium mycolates (8 mg, around 6 µmol) were added to 1-*O*-(4 methylbenzenesulfonyl)-2,3-*O,O*-isopropylidene-sn-glycerol (2 mg, 7 µmol) in anhydrous dimethylformamide (0.150 ml) and stirred for 3 days at 135 °C. The reaction mixture was acidified by 1M hydrochloric acid and extracted by diethyl ether. Ether extract was then washed with water, dried on magnesium sulphate and concentrated. Chromatography on silica gel (elution with petroleum ether / diethyl ether 5/1, v/v) gave the product (around 4 mg, 2.9 µmol). In the same way, 3-*O*-mycoloyl-1,2-*O*,*O*-isopropylidene-sn-glycerol was obtained from 3-*O*-(4 methylbenzenesulfonyl)-1,2-*O*,*O*-isopropylidene-sn-glycerol.

*1-*O*-Mycoloyl-sn-glycerol (GroMM)* (Sowden et al. (1942) J Am Chem Soc 42, 1291-3) : 100 µl of concentrated hydrochloric acid (d=1.017) were added to 1-*O-*Mycoloyl-2,3-*O*,*O*- isopropylidene-sn-glycerol (4 mg, 2.9 µmol ) dissolved in diethyl ether (0.1 ml). After 10 min of stirring at room temperature, water was added to the reaction mixture and the product (3 mg, 2.3 µmol) was extracted with diethyl ether. The ether extract was dried on magnesium sulphate and concentrated. Supplemental purification was not necessary. 3-O-mycoloyl-sn-glycerol was also obtained using 3-*O*-mycoloyl-1,2-*O,O*-isopropylidene-sn-glycerol.

**MALDI-*Tof*-MS.** Analysis by MALDI-*Tof*-MS were performed on a 4700 Proteomics Analyser (with Tof-Tof Optics, Applied Biosystems) using the reflectron mode. Ionization was effected by irradiation with pulsed UV light (355 nm) from an Nd:YAG laser. GroMM samples were analyzed by the instrument operating at 20 kV in the positive ion mode using an extraction delay time set at 20 ns. Typically, spectra from 100 to 250 laser shots were summed to obtain the final spectrum. The HABA (2-[4-hydroxy-phenylazo]-benzoic acid) matrix (Sigma-Aldrich) was used at a concentration of -10 mg/ml in ethanol/water (1:1, v/v). Then, 0.5 µl sample solution and 0.5 µl of the matrix solution were deposited on the target, mixed with a micropipette and dried under a gentle stream of warm air. The measurements were externally calibrated at two points with mycobacterial PIM.

**Nuclear Magnetic Resonance (NMR) Analysis.** NMR spectra were recorded with an Avance DMX500 spectrometer (Bruker) equipped with an Origin 200 SGI using Xwinnmx 2.6. GroMM was dissolved in CDCl₃-CD₃OD, (9:1, v/v) and analyzed in 200 x 5 mm 535-PP NMR tubes at 298 K. Proton chemical shifts are expressed in parts per million downfield from the signal of the chloroform (δ_{H}/TMS 7.27). All the details concerning correlation spectroscopy and homonuclear Hartmann-Hahn spectroscopy sequences used and experimental procedures were as previously described Gilleron et al. (2003) J Biol Chem 278, 29880-9.

**Recognition of GroMM by Lymphocytes from Tuberculosis Patients and Healthy Donors.** PBMCs were purified from blood of healthy donors and tuberculosis patients after informed consent was given. All patients included in this study suffered from culture proven pulmonary tuberculosis and were undergoing treatment with anti-tuberculosis drugs for 2 to 8 weeks at the time of blood donation. PBMCs (10⁵/well) were cultured with autologous DCs (2x10⁴/well) in the presence or absence of 10 µg/ml GroMM. After 6 days supernatants were collected and tested for the presence of IFN-γ by sandwich ELISA. PBMCs were kept at 37°C in supplemented RPMI 1640 medium (see above) during the 2 days of autologous monocytes differentiation to DCs. In parallel with GroMM PBMCs were stimulated with 10 µg/ml PPD (Chiron) and recombinant ESAT6 (Lionex). Healthy donors that responded by releasing at least 250 pg/ml IFN-γ in response to PPD were scored as BCG immunized. Donors that responded to both antigens were considered as latently *M*. *tuberculosis* infected. Those that did not respond to PPD were scored PPD-negative. For individual experiments, a cocktail of CD1b-and CD1c-blocking antibodies (BCD1b3.1 and F10 - 10µg/ml each) was added 30 min prior to GroMM to block CD1 presentation.

### Results

### Characterization of lipid-reactive T cells clones

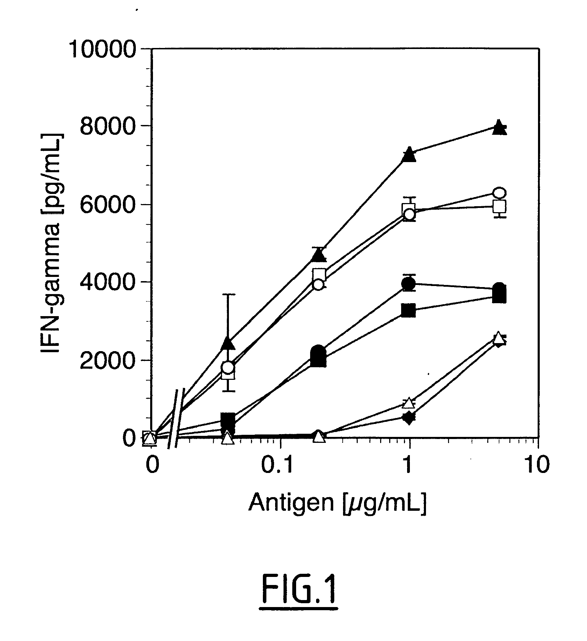

In order to identify the *M*. *tuberculosis* CD1-restricted T cell lipid antigen repertoire, a strategy was developed based on the generation of T cell lines stimulated by three different types of crude mycobacterial lipid fractions, according to their polarity and charge (Gilleron et al. (2004) J Exp Med 199, 649-59). From these lines, derived from PBMC of one highly PPD-reactive healthy donor, T cell clones were established by limiting dilution and 10 individual clones which were found CD1-restricted, were further investigated. One of these clones, called Z5B71, which is CD4⁺ was investigated. It was obtained against a fraction enriched in phosphatidyl-*myo*-inositol dimannosides (PIM₂), obtained from *M*. *bovis* BCG cell wall extraction. This clone reacts to lipidic extracts from different mycobacterial species (*M*. *bovis* BCG, *M*. *smegmatis*, *M*. *gastri*, *M*. *marinum*, *M*. *fortuitum*, *M*. *xenopi*, *M*. *kansasii* and *M*. *tuberculosis* H37Ra) and from *Corynebacterium glutamicum* and *Nocardia asteroides* (Fig. 1). The recognized lipid antigen thus is apparently widely distributed in the *Mycobacterium* genus, and is also present in bacteria of the CMN group. Interestingly, among the tested lipidic extracts, that from *M*. *bovis* BCG always appeared the most stimulatory, suggesting that this antigen is more abundant in these bacteria.

### Purification of the lipid antigen stimulating Z5B71 cells

Purification of the lipid antigen was performed using *M. bovis* BCG lipidic extract because it showed the strongest stimulatory activity and also because the diversity of glycolipids in the cell envelope of this species is lower than in that of *M*. *tuberculosis.* Lipidic extract was partitioned by acetone precipitation, a solvent known to precipitate phospholipids. Both "acetone-soluble" and "acetone-insoluble" fractions (Fig. 11A) stimulated the Z5B71 T cell clone (Fig. 11B), suggesting that, i) the antigen does not preferentially partition in one fraction or ii) different antigens bearing a common epitope are recognized by these T cells. Since the stronger reactivity was observed with the "acetone-insoluble" fraction, this fraction was further precipitated by methanol. Z5B71 T cells again reacted to both phases (Fig. 11 C), although the response was higher with the "methanol-insoluble" fraction.

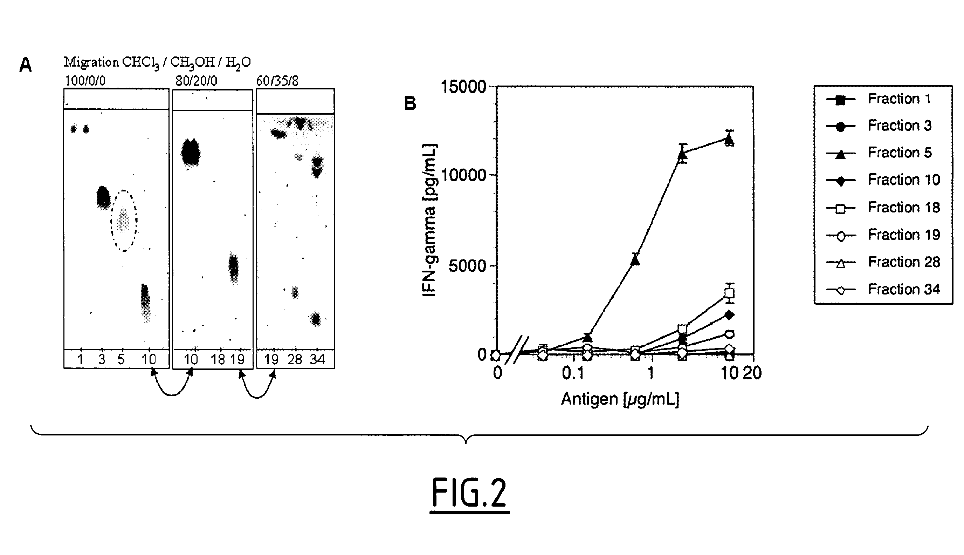

"Methanol-insoluble" fraction was then fractionated on a silica gel column eluted by chloroform containing increasing amounts of methanol (Fig. 11A). Z5B71 T cells were stimulated by fractions 5 to 9 (Fig. 2B), eluted by chloroform or chloroform/methanol, 95/5 (v/v). When analyzed by TLC, the active fractions appeared to contain a homogenous product (Fig. 2A). The fractions 5 to 9 were pooled and the resulting fraction was further analyzed by MALDI-*Tof*-MS and NMR.

### Chemical characterization of a novel mycobacterial lipid antigen

The positive mode MALDI mass spectrum showed a complex pattern of peaks assigned to cationized sodiated molecular ions [M+Na]⁺ (Fig. 3A). The main peaks in the massif are distant of 28 mass units, suggesting differences of two methylenic units between the different molecular species, possibly as result of heterogeneity in fatty acid length. To confirm this assumption, the product was hydrolyzed under alkaline conditions and the fatty acids were analyzed by GC (data not shown) and MALDI-*Tof*-MS (Fig. 3B). The positive mode MALDI spectrum highlighted a series of peaks characteristic of mycolic acids (Laval et al., (2001) Anal Chem. 73(18):4537-44). According to the presence of various chemical functions on the meromycolic chain, mycolic acids are subdivided into α-mycolic acids, methoxymycolic and ketomycolic acids (Daffe and Draper (1998) Adv. Microb. Physiol 39:131-203). *M*. *tuberculosis* contains a ratio α-mycolic acids / oxygenated mycolic acids (methoxymycolic acid + ketomycolic acid) of 1/1, while *M*. *bovis* BCG contains predominantly ketomycolic acids (Watanabe et al. (2001) Microbiology 147, 1825-37). In agreement with these data, peaks at m/z 1232, 1260, 1274, 1288 and 1302 (Fig. 3B) were assigned to [M+Na]⁺ forms of ketomycolic acids (Fig. 3C).

The mass difference (Δ 74 amu) between the native product and the resulting mycolic acid after alkaline treatment suggested that the mycolic acid esterifies a glycerol residue. The number of alcohol functions on the molecule was proved by peracetylation of the molecule, and analysis of the reaction products by MALDI MS (not shown). The displacement of 126 mass unit proved the presence of three alcohol functions. Then, the assignment of the novel antigen to glycerol monomycolate (GroMM) was further supported by 1D ¹H NMR analysis (Fig. 4A). The resonances typical of mycolic acids were assigned according to published studies (Watanabe et al. (2001) Microbiology 147, 1825-37; Schroeder et al. (2001) Bioorg Chem 29, 164-77 ; Quemard et al. (1997) Eur J Biochem 250, 758-63. Some of the protons of the meromycolic chain could be easily interpreted: protons d (δ 2.31) and e (δ 3.53, multiplet), which correlate together on the COSY spectrum (not shown), were assigned to the methyne carrying the α-chain and the β-hydroxyl groups, respectively. The protons j (δ 2.41, multiplet) and k (δ 0.92, doublet) which also correlate together on the COSY spectrum were assigned to the methyne carrying a methyl group and to this methyl, respectively in the distal portion of the meromycolic chain. The signal / (δ 0.75, triplet) is assigned to the protons of the terminal methyl groups of the α-chain and the meromycolic chain. The multiplets at δ -0.46, δ 0.44 and δ 0.52 (labelled *h*), that correlate together on the COSY spectrum, are the characteristic signals of the cyclopropanic function protons. Signals around 5 ppm (*g* at δ 5.16 and ***f*** at δ 4.83) correspond to olefinic protons, indicating that a small proportion of the mycolic acids contain ethylenic groups. Protons a (δ 4.11, 4.06 and 3.99), *b* (δ 3.75) and *c* (δ 3.47), which correlate together on the HOHAHA spectrum (not shown), were assigned to glycerol protons. However, protons a were divided into two independent systems, *a1* (δ 4.11 and 3.99) and a2 (δ 4.06), whose exact assignment was made possible by NMR analysis of synthetic GroMM analogues.

### Hemi-synthetic GroMM (sGroMM) is immunogenic

In order to confirm the structure of the antigen, GroMM analogues were chemically synthesized (Fig. 12), using the mycolic acids isolated from *M*. *tuberculosis* H37Rv. *M*. *tuberculosis* H37Rv mycolic acid analysis by MALDI-MS (Fig. 5B) showed [M+Na]⁺ forms of methoxymycolic acids at m/z 1248, 1276, 1304 and 1332 as the major species. Ions corresponding to ketomycolic acids (peaks at m/z 1260, 1288, 1302 and 1316) and α-mycolic acids (peaks at m/z 1160, 1188 and 1216) were also observed in a lower abundance, as previously described Laval et al. (2001) Anal Chem 73, 4537-44.

The chemical synthesis was started using (R)- and (S)-isopropylidene-glycerol, activated by p-toluene-sulfonyl (Sowden et al. (1942) J Am Chem Soc 42, 1291-3) (Fig. 12). The esterification of glycerol by mycolic acids was realized by heating (R) or (S)-isopropylidene-glycerol in the presence of potassium mycolates (Defaye et al. (1956) Bull Soc Chim Biol (Paris) 38, 1301-4). Both isopropylidene-mycoloyl-glycerol isomers were then deprotected under acidic conditions giving synthetic (R)- or (S)-GroMM (s(R)-GroMM and s(S)-GroMM, respectively). The MALDI mass spectra of both sGroMM (Fig. 5A) were in agreement with the expected mass distribution of GroMM bearing *M*. *tuberculosis* H37Rv mycolic acids (Fig. 5C). Both sGroMM were then analyzed by 1D ¹H NMR (Fig. 6B and C). The analysis of the 1-O-mycoloyl-sn-glycerol (s(S)-GroMM) showed *a* protons (designed *a1*) at 4.05 ppm (Fig. 6B), while the analysis of the 3-O-mycoloyl-*sn-*glycerol (s(R)-GroMM) showed a protons (designed a2) at 4.00 and 4.12 ppm (Fig. 6C). These data allowed then explaining the complexity of the massif of glycerol protons *a* on the spectrum of the natural GroMM, which corresponds to a mixture of both isomers (Fig.6D). From the intensity of *a1* and *a2* resonances (Fig. 6D), it can be deduced that natural GroMM is composed of both stereoisomers in similar proportions.

The s(R)-GroMM stimulated the Z5B71 T cells more efficiently than the s(S)-GroMM, while the purified, natural compound has an intermediate activity (Fig. 7). Taken together, both the NMR analysis and T cell reactivity confirmed the structure of the natural antigen.

### GroMM is presented by CD1b and does not require the presence of CD1e

The CD1 restriction element of GroMM was investigated by stimulating the T-cell clone using a panel of CD1-transfected APCs. Only CD1b-expressing cells were able to stimulate T cells (Fig. 8A). Furthermore, the CD1 b restriction was further supported by blocking experiments, in which only anti-CD1b mAbs were capable of inhibiting the reactivity of the clone (Fig. 8B). As it was previously demonstrated that CD1e could assist the presentation of mycobacterial lipids (de la Salle et al., (2005) Science 310(5752):1321-4), the antigenicity of GroMM was also tested using APCs expressing both CD1b and CD1e or only CD1b, in order to investigate the possible participation of CD1e. Both APCs induced very similar T cell response (Fig. 8C), thus excluding the participation of CD1e in the presentation of GroMM.

### GroMM immunogenicity in infected cells

An important issue is whether mycobacterial lipid antigens maintain their antigenicity during infection, i.e. when they are released from the cell wall of the bacteria resident in the phagosome. This was investigated by using DCs which were pulsed either with heat-killed *M*. *tuberculosis,* or which were infected with living bacteria. DCs pulsed with heat-killed bacteria were used to stimulate three T cell clones recognizing three different *M*. *tuberculosis* lipidic antigens, namely GroMM (Z5B71), Ac₂SGL (Z4B27) and PIM₆ (Z5B11). The Ac₂SGL and PIM₆-reactive cells (de la Salle et al. (2005) Science 310, 1321-4 ; Gilleron et al. (2004) J Exp Med 199, 649-59) were previously described. High doses of heat-killed bacteria (100 µg/ml) induced a strong T cell response (Fig. 9A-C), confirming that all three lipids become available for CD1b loading when associated with the bacterial cell wall. GroMM was highly immunogenic also when APCs were infected with living *M*. *tuberculosis,* demonstrating that GroMM are presented by DCs during infection (Fig. 9D).

### T cells from PPD-positive healthy donors recognize GroMM

To directly test whether T cells in the peripheral blood of human donors recognize the novel lipid compound, blood samples were collected from tuberculosis patients (n=11), PPD-negative (n=10) and BCG immunized (n=22) and latently *M*. *tuberculosis* infected (n=19) healthy donors and measured IFN-γ release after GroMM stimulation. Significant responses were solely observed in BCG immunized or latently infected, healthy donors (with a mean average of 73 pg/ml and 117 pg/ml, respectively; Fig. 10A). Although PBMCs from tuberculosis patients vigorously responded to PPD (data not shown), GroMM induced no IFN-γ secretion above background levels in this donor cohort. Similarly, no response was observed in naïve, PPD-negative donors. GroMM induced IFN-γ secretion was CD1 restricted, because blocking antibodies directed against type 1 CD1 molecules abrogated the reactivity (Fig. 10B) in contrast to MHC-I blocking antibodies (data not shown). The presence of GroMM specific, CD1-restricted T cells in the peripheral blood of human donors therefore represents a mycobacteria induced T cell memory. However, this applies only for healthy donors, while patients with active tuberculosis failed to recognize GroMM.

### Increased activity of the reduced compounds

To test the activity of the reduced compounds, the stimulation of the Z5B71 T cell clone by measuring IFN-γ release of GroMM and reduced GroMM was measured. The results shown in Fig. 13 illustrate the increased activity of the reduced GroMM.

### Synergistic activity of combinations of mycobacterial GroMM with lipids

To test whether lipids would potentialize the acitivity of mycobacterial GroMM, the stimulation of the Z5B71 T cell clone by measuring IFN-□release of both agents alone or in combination was measured for various mycobacterial lipids (A) or phospholipids (B). The results shown in Fig. 14 illustrate the synergistic effect.

## Claims

1. A pharmaceutical composition comprising at least one compound of formula (I): 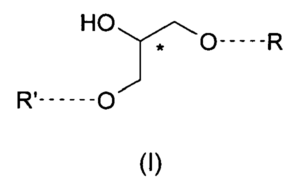 wherein
R is a residue of a α-alkylated, β-hydroxylated carboxylic acid comprising 20 to 90 carbon atoms;
R' represents a hydrogen atom or acyl group (-C(=O)-Alkyl) where Alkyl comprises 1 to 10 carbon atoms; and
* denotes an asymmetric carbon atom;
and its enantiomers, diastereoisomers, mixtures thereof.

2. The pharmaceutical composition according to claim 1, wherein R is of formula: 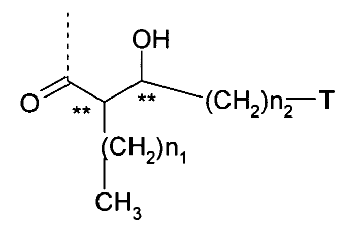 where (I) is attached to R through ---- ; and
T represents a methyl group or a branched or linear C10-C60 hydrocarbon chain which optionally comprises one or more double bond(s), -(C=O)- group, cyclopropyl and/or epoxy, and/or is optionally substituted with one or more groups chosen from OH, OMethyl;
** denotes an asymmetric carbon;
n1, n2 identical or different, independently represent an integer.

3. The pharmaceutical composition according to claim 2, wherein n1 is an integer comprised between 5 and 40.

4. The pharmaceutical composition according to claim 2 or 3, wherein n2 is an integer comprised between 5 and 30.

5. The pharmaceutical composition according to claim 2, 3 or 4, wherein T is chosen from:
- a methyl group; or
- a branched or linear C10-C60, which optionally comprises one to five double bond(s); or
- a group of formula: 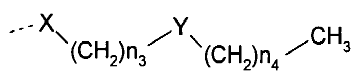
where
X is chosen from : 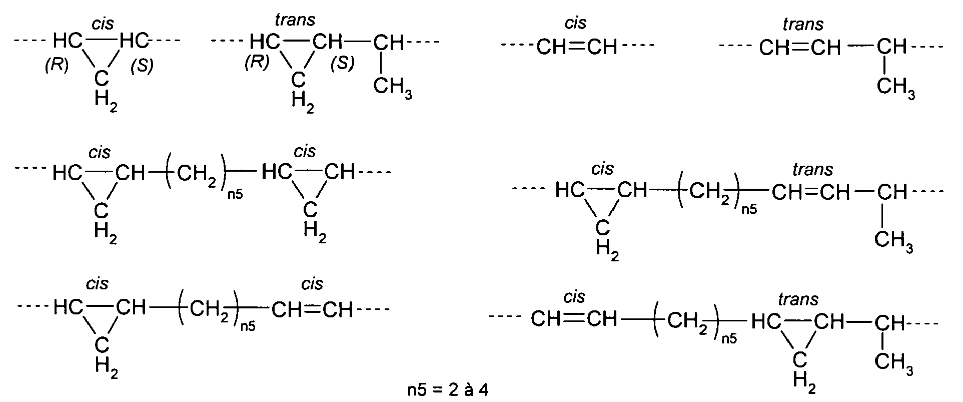 Y is chosen from: 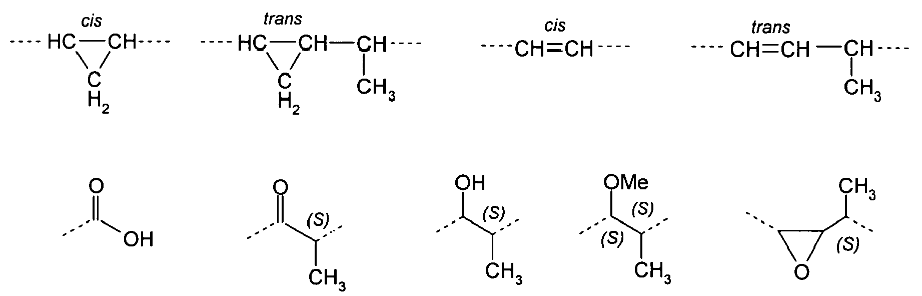 and n3, n4 identical or different, independently represent an integer comprised between 5 and 30.

6. The pharmaceutical composition according to claim 5, wherein n3 represents an integer comprised between 12 to 16.

7. The pharmaceutical composition according to claim 5 or 6, wherein n4 is chosen from 11, 13, 15, 17, 18 and 19.

8. The composition according to anyone of the preceding claims comprising at least two different compounds of formula (I) such as defined in anyone of the preceding claims.

9. The composition according to anyone of the preceding claims comprising a mixture of compounds of formula (I) as defined in anyone of the preceding claims.

10. The pharmaceutical composition according to anyone of the preceding claims **characterized in that** it is presented in a form intended for administration by oral or injectable route.

11. The pharmaceutical composition according to anyone of the preceding claims, **characterized in that** it comprises one or more other products useful for the treatment or the prophylaxis of tuberculosis.

12. The pharmaceutical composition according to claim 11, wherein said products useful for the treatment or the prophylaxis of tuberculosis are chosen from BCG or mycobacterial lipids.

13. The pharmaceutical composition according to claim 11, wherein said mycobacterial lipids are chosen from the group consisting in mycobacterial phospholipids, mycobacterial lipoglycans, mycobacterial glycolipids and phospholipids.

14. The pharmaceutical composition according to claim 11, 12 or 13 as a combined preparation for simultaneous, separate or sequential use in the treatment or the prophylaxis of tuberculosis.

15. The pharmaceutical composition according to anyone of the preceding claims which is a vaccine.

16. A compound of formula (I) 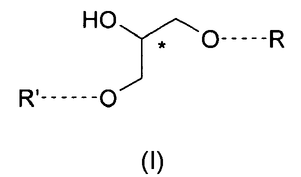 wherein:
R' represents a hydrogen atom or acyl group (-C(=O)-Alkyl) where Alkyl comprises 1 to 10 carbon atoms;
* denotes an asymmetric carbon atom; and
R is of formula: 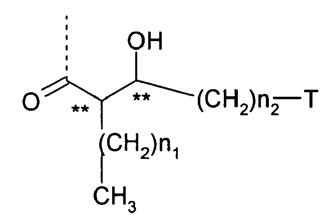 where (I) is attached to R through ---- ;
** denotes an asymmetric carbon;
n1, n2 identical or different, independently represent an integer; and
T is a group of formula: 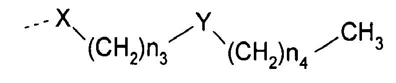
where:
n3, n4 identical or different, independently represent an integer comprised between 5 and 30
X is chosen from : 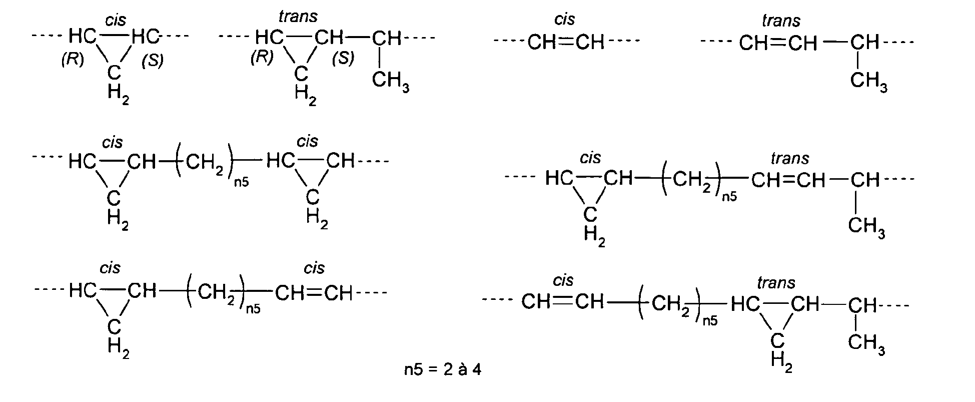
and Y is 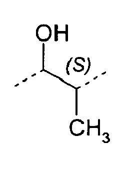 and its enantiomers, diastereoisomers, mixtures thereof.

17. A compound of formula (I) 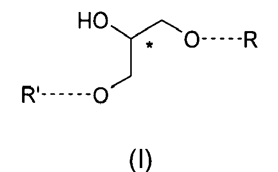 wherein:
R is a residue of a α-alkylated, β-hydroxylated carboxylic acid comprising 20 to 90 carbon atoms;
R' represents an acyl group (-C(=O)-Alkyl) where Alkyl comprises 1 to 10 carbon atoms; and
* denotes an asymmetric carbon atom;
and its enantiomers, diastereoisomers, mixtures thereof.

18. The compound according to claim 17, wherein R is of formula: 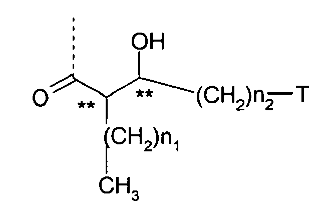 where (I) is attached to R through ---- ; and
T represents a methyl group or a branched or linear C10-C60 hydrocarbon chain which optionally comprises one or more double bond(s), -(C=O)- group, cyclopropyl and/or epoxy, and/or is optionally substituted with one or more groups chosen from OH, OMethyl;
** denotes an asymmetric carbon;
n1, n2 identical or different, independently represent an integer.

19. The compound according to claim 16, 17 or 18, wherein n1 is an integer comprised between 5 and 40 and n2 is an integer comprised between 5 and 30.

20. The compound according to claim 18 or 19, wherein T is chosen from :
- a methyl group; or
- a branched or linear C10-C60, which optionally comprises one to five double bond(s); or
- a group of formula: 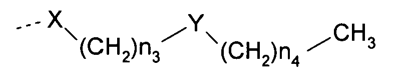
where
X is chosen from: 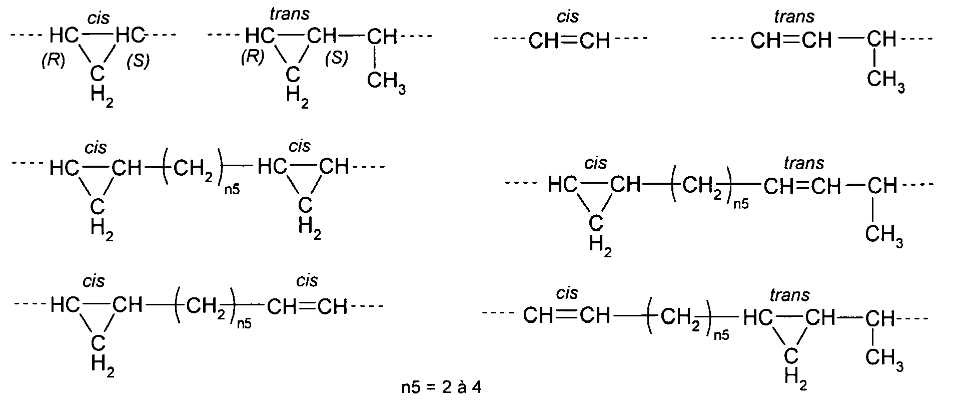 Y is chisen from: 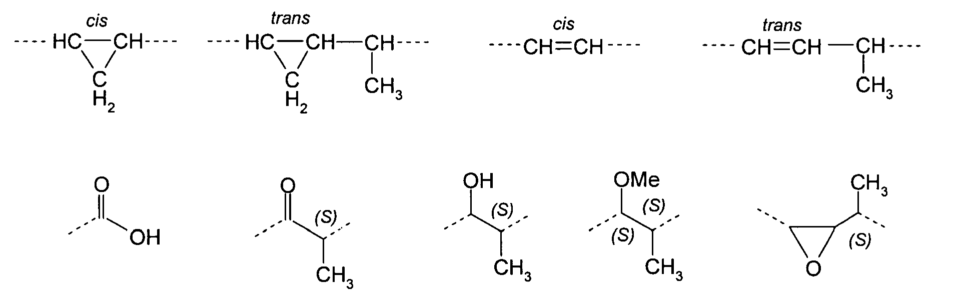 and n3, n4 identical or different, independently represent an integer comprised between 5 and 30.

21. The compound according to claim 16 or 20, wherein n3 represents an integer comprised between 12 to 16 and n4 is chosen from 11, 13, 15, 17, 18 and 19.

22. A compound as defined in anyone of claims 1 to 15, **characterized in that** in formula (I) the C* exhibits the *(R)* configuration.

23. Use of at least one compound of formula (I) as defined in anyone of claims 1 to 22 for the preparation of a medicament intended for the treatment or the prophylaxis of tuberculosis.

24. A compound as defined in anyone of claims 1 to 22 as a medicament for the treatment or the prophylaxis of tuberculosis.

25. Use of at least one compound as defined in anyone of claims 1 to 22 as an immune reaction activator.

26. Use of at least one compound as defined in anyone of claims 1 to 22 to induce the activation of T lymphocytes.

27. Use of at least one compound as defined in anyone of claims 1 to 22 to induce the production of IFN-γ, TNF-α, IL-2, IL-4, or GM-CSF.

28. A process for generating a T cell clone specific to the compound of formula (I) as defined in anyone of claims 1 to 22, **characterized in that** it comprises the following stages:
1) incubating antigen presenting cells (APCs), notably dendritic cells, with an actinomycete lipidic preparation, with the exception of *Mycobacterium tuberculosis* lipidic preparation, or a compound of formula (I),
2) contacting peripheral blood mononuclear cells with the envelope antigen loaded APCs to obtain proliferating T cells,
3) cloning proliferating T cells by limiting dilution and selecting the clone releasing a molecule selected from the group comprising cytokines such as IFN-γ, TNF-α, GM-CSF, IL-2 or IL-4 when contacted by a compound of formula (I).

29. The process according to claim 28, wherein in step 1), the actinomycete lipidic preparation is a *Mycobacterium bovis* BCG lipidic preparation.

30. T cell clones obtainable by the process according to claim 29.

31. A process for screening products **characterized in that** it comprises the following stages:
- contacting dendritic cells loaded with the product to screen with the T cell clones according to claim 30,
- detecting a molecule selected from the group comprising cytokines, such as IFN-γ, TNF-α, GM-CSF, IL-2 or IL-4, released by the T cell clones.

32. A process for the extraction of compounds as defined in anyone of claims 1 to 22 from actinomycetes, **characterized in that** it comprises the following stages:
a. treatment of the bacteria with a mixture of methanol and chloroform to obtain a chloroform/methanol extract,
b. concentration of the chloroform/methanol extract followed by its partition between a chloroform phase (herein called the lipidic fraction) and an aqueous phase,
c. taking off the lipidic fraction.

33. The process according to claim 32, which further comprises the following additional steps:
d. evaporation of most of the chloroform, followed by addition of acetone thereto to obtain a precipitate and a soluble acetone phase,
e. taking of the acetone insoluble phase followed by its concentration, followed by addition of methanol to obtain a precipitate and a methanol insoluble phase,
f. taking of the methanol insoluble phase, followed by its concentration and application of the concentrated methanol insoluble phase on a silicic acid column,
g. elution of a fraction from the above-mentioned silicic acid column by chloroform, methanol and mixtures thereof, said fraction corresponding to a composition as defined above,
h. if necessary, purification of the fraction eluted from the silicic acid column to obtain different preparations respectively containing substantially only one of the compounds as defined above.

34. A composition obtainable by the process according to claim 32 or 33.
